# EUROPEAN PATENT SPECIFICATION

(11) **EP 3 188 702 B1**
(45) Date of publication and mention of the grant of the patent: **20.11.2019**
(21) Application number: 15770978.3
(22) Date of filing: 02.09.2015
(51) Int. Cl.: A61F 13/491, A61F 13/84, A61B 5/145, A61F 13/42, A61F 13/471, G01N 33/52

(54) **DEVICE FOR HOLDING AN INDICATOR**
VORRICHTUNG ZUM HALTEN EINES INDIKATORS
DISPOSITIF DE MAINTIEN D'UN INDICATEUR

(30) Priority: 03.09.2014 SE 1451023
(43) Date of publication of application: 12.07.2017
(73) Proprietor: Danell, Nils, 151 37 Södertälje (SE)
(72) Inventor: Danell, Nils, 151 37 Södertälje (SE)
(74) Representative: Brann AB
(86) International application number: PCT/SE2015/050922
(87) International publication number: WO 2016/036302

(56) References cited:
- WO-A1-01/50996
- WO-A1-2011/162657
- US-A- 5 383 867
- US-A1- 2003 166 293

## Description

### Field of the invention

The present invention relates to medical devices and in particular to a device for holding in place an indicator for detection of disease in the urogenital system.

### Background of the invention

Today, diseases in the urogenital system and urinary tract are discovered late, leading to suffering for individual patients and significant costs for society. A late discovered infection in the urinary tract may necessitate treatment with antibiotics involving medical practitioners and may also lead to serious complications. Early discovery reduces suffering, risk for complications, as well as healthcare costs.

Indicators for detection of gaseous amines produced by microorganisms are known in the art and available from e.g. Siemens Healthcare Diagnostics AB (Upplands Väsby, Sweden).

In the following some patent documents in the general field of personal care products are briefly discussed.

WO2005/061013 discloses an absorbent personal care product comprising an indicator with an amine sensitive dye, placed in the product such that the dye deposit is visible to the unaided eye.

US-2005/0124072 relates to a personal care product with visual indicator of vaginitis.

WO-2011/162657 discloses a male incontinence protector to solve the problem of securely and comfortably retaining the protector in close contact with the body of the wearer.

WO 01/50996 A1 discloses a device for gauging the levels of pH in the interiors of articles such as diapers, incontinence garments and the like. It comprises pH sensing means and means for displaying the pH on the outside of the device.

However, one drawback of the indicator disclosed in WO-2005/061013, and also in US-2005/0124072, is that measurements may be erroneous, due to that gaseous amines from the wearer's anus may influence the measurements, resulting in a detection of a suspected disease in the urogenital system.

Thus, the object of the present invention is to achieve an improved device for detection of disease in the urogenital system giving reliable measurement results, and that is easy to use.

### Summary of the invention

The present invention relates to a device according to the independent claim. Preferred embodiments are set forth in the dependent claims.

The present invention provides means for early detection of disease in the urogenital system by providing a device adapted to hold an indicator of amine metabolites produced by microorganisms and affected tissues in place in close proximity to orifices of the human urogenital system, such that gaseous amines from the anus do not influence the measurements. This is essentially achieved by the triangular shape of the device. Particularly the present invention relates to a device provided with an indicator pocket into which an indicator may be removably arranged. The device is specifically advantageous in that it is easy to use, and the resulting measurements are reliable as only gaseous amines from urogenital orifices may reach the active part of the indicator.

### Short description of the appended drawings

Figures 1A-1C show various views of one embodiment of the invention.
Figure 2 shows an embodiment of the invention for use by men.

### Detailed description of preferred embodiments of the invention

The invention will now be described in detail with references to the appended drawings. Throughout the figures the same or similar items have the same reference signs.

The invention generally relates to a device for detection of gaseous amines originating from disease in the human urogenital system, said device comprising an inner surface for facing the user and and outer surface for facing away from the user, wherein said device comprises an indicator of gaseous amines, and is made of a flexible material of a shape adapted to cover orifices of the human urogenital system but not the anus.

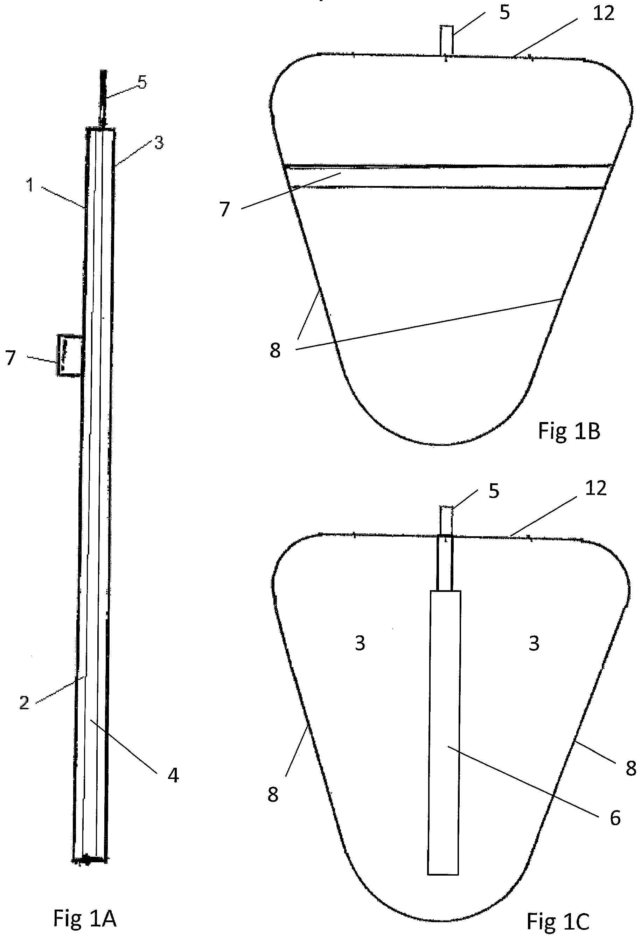

Figure 1A shows a side view of one embodiment of the device according to the invention. The device is made of a flexible material that may be worn between the genital area and a pair of underpants of a user. Examples of flexible materials are woven and non-woven materials. The device preferably does not contain chlorhexidine or other disinfectants. The device has an outer surface 1 and an inner surface 3. Figure 1B is a view showing the outer surface side of the device, and Figure 1C is a view showing the inner surface side of the device.

The device comprises an indicator of gaseous amines 5 that is introduced into the device. According to the invention the indicator 5 is removably attached to the device by providing it in an indicator pocket 6 on the inner surface 3 of the device. The indicator has an elongated shape, being essentially flat, and is made from a soft flexible material having a structural integrity that ensures that it may be easily introduced into the pocket 6. The indicator comprises an active part including an amine sensitive dye, and that the active part of the indicator is positioned such that amine sensitive dye is optimally located to detect amine metabolites produced by microorganisms and affected tissues in place, e.g. in close proximity to orifices of the human urogenital system. In one embodiment the active part is provided close to the distal part of the indicator, being the part that is furthest away from the opening of the indicator pocket when the indicator is correctly inserted into the indicator pocket. The active part is preferably located at both sides of the indicator, which is important in order to secure that a reliable indication may be obtained, i.e. that a part of the active part faces the skin of the patient.

The indicator pocket has a length that corresponds to at least 75% of the length of the indicator. This is important in order for the indicator to be held properly in place during use. The width of the indicator pocket is slightly larger than the width of the indicator. The orientation of the indicator pocket is preferably essentially such that, when the device is arranged to be used, the pocket runs along a vertical symmetry line of the user's body. Thereby the indicator will be arranged in an optimal position in relation to the urogenital orifice of the body.

The device may comprise several layers. It is essential that the layer constituting the inner surface 3 and any further layer between the inner surface 3 and the indicator 5 is permeable to the gaseous amines that are to be detected by the indicator 5. Further layers may include a liquid barrier layer 2 and a moisture absorbing layer 4.

The liquid barrier layer 2 may be any liquid impermeable layer that is sufficiently flexible for inclusion in the device without causing discomfort to the user. Examples of such liquid barrier layers are plastic, e.g. a polypropylene or polyethylene film, or hydrophobic woven and non-woven materials.

The moisture absorbing layer 4 is, if present, preferably made of cellulose such as fluff.

In a preferred embodiment of the invention, the device does not comprise an absorbing layer 4.

In one embodiment of the invention, the device is fitted with a band 7 across the outer surface 1 of the device, wherein said band 7 is arranged to allow a user to hold the device by insertion of a hand between the band and rest of the device, without touching the indicator 5.

In order to prevent the device from sliding towards the anus during use, the shape of the device is generally triangular, as shown in figures 1B and 1C, so that the two edges 8, having equal lengths, of the device abut the groin of the wearer thereby preventing undesired movement. The triangle is an isosceles triangle that has two sides of equal length. More particularly, the device comprises an upper edge 12 being the side of the triangle to be positioned essentially horizontally and upward and being closest to the stomach of the patient.

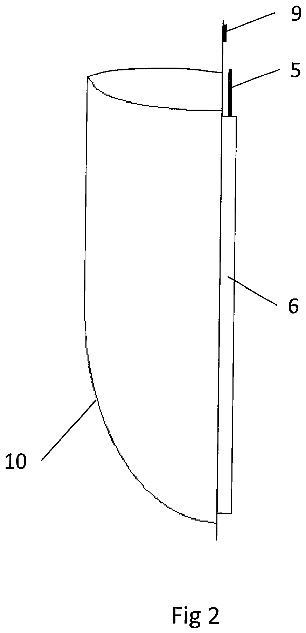

The device may also be provided with means 9, as shown in Figure 2, for removably attaching the device to an underpant in order to prevent undesired movement. Such means may be i.a. an adhesive patch, velcro, or a hook or catch.

In one embodiment of the invention, the device is adapted for male users. The device then includes a pocket 10 wherein the penis may be put. The pocket 10 is at the side of the device that faces the body, and is preferably made from a gas impermeable material in order to further prevent gas contamination of the indicator 5 by gaseous amines from the anus. However, no gas impermeable material is provided in the part of the pocket 10 close to the indicator pocket 6, in particular close to the lower part of the pocket 10 where the urogenital orifice is located during use.

The indicator 5 may comprise amine sensitive chemichromic dyes that change color in the presence of amines, such as trimethylamine, putrescine and cadaverine commonly produced in infections in the urogenital system. Such indicators are available from inter alia Siemens Healthcare Diagnostics AB (Upplands Väsby, Sweden). One class of such chemichromic dyes that is particularly useful is arylmethane dyes, such as diarylmethanes, triarylmethanes, and the like.

The indicator is preferably provided as a separate item where the active part is protected e.g. by a protective strip that is removed in order to activate the chemichromic dye. The indicator is then inserted into the indicator pocket and kept there during a time period, e.g. a couple of hours. After use the device is removed and the indicator is removed from the indicator pocket. The color of the active part of the indicator is then compared to a color key provided e.g. at a piece of paper, in connection with an application program of a mobile phone, or by another mean, to determine if any amines have been measured.

The present invention is not limited to the above-described preferred embodiments. Various alternatives, modifications and equivalents may be used. Therefore, the above embodiments should not be taken as limiting the scope of the invention, which is defined by the appended claims.

## Claims

1. Device for detection of gaseous amines originating from disease in the human urogenital system, said device comprising an outer surface (1) and an inner surface (3) for facing the user and for facing away from the user, respectively, **characterized in that**
said device comprises an indicator (5) configured to detect gaseous amines, comprising amine sensitive chemichromic dyes that change color in the presence of amines;
said device is made of a flexible material of a shape adapted to cover orifices of the human urogenital system but not the anus, the device having a liquid barrier (2) near the outer surface and the inner surface (3) is permeable to the gaseous amines that are to be detected by the indicator (5); wherein
the shape of the device is generally triangular so that edges (8) of the device abut the groins of the wearer thereby preventing undesired movement;
the indicator is removably placeable in an indicator pocket (6) provided in the gas permeable layer of the inner surface (3); and
the indicator has an elongated shape and is essentially flat, and is made from a soft flexible material having a structural integrity that ensures that it may be easily introduced into the pocket (6).

2. The device according to claim 1, wherein the chemichromic dye is sensitive to amines selected from the group consisting of trimethylamine, putrescine and cadaverine.

3. The device according to claim 1, wherein chemichromic dyes are selected from the group consisting of arylmethane dyes.

4. The device according to claim 3, wherein the dyes are selected from the group consisting of diarylmethanes and triarylmethanes.

5. The device according to any of claims 1-4, not comprising a moisture absorbing layer.

6. The device according to any of claims 1-4, comprising a moisture absorbing layer (4) between the inner surface (3) and the liquid barrier layer (2).

7. The device according to any of claims 1-6, further comprising a band (7) across the outer surface of the device, wherein said band is arranged to allow a user to hold the device by insertion of a hand between the band and rest of the device, without touching the indicator.

8. The device according to any one of claims 1-7, further comprising means (9) for removably attaching the device to an underpant.

9. The device according to any one of claims 1-8, further comprising a pocket (10) attached to the inner surface, for holding the penis of the user.

## Patentansprüche

1. Vorrichtung zum Erfassen von gasförmigen Aminen, die von Erkrankung im menschlichen urogenitalen System ausgehen, wobei die Vorrichtung eine Außenfläche (1) und eine Innenfläche (3) umfasst, die jeweils dem Benutzer zugewandt und von dem Benutzer abgewandt sind, **dadurch gekennzeichnet, dass**
die Vorrichtung einen Indikator (5) umfasst, der konfiguriert ist, um gasförmige Amine zu erfassen, umfassend aminempfindliche chemichrome Farbstoffe, die in der Gegenwart von Aminen die Farbe ändern;
wobei die Vorrichtung aus einem flexiblen Material einer Form gefertigt ist, die ausgelegt ist, um Öffnungen des menschlichen urogenitalen Systems zu bedecken, aber nicht den Anus, wobei die Vorrichtung eine Flüssigkeitsbarriere (2) nahe der Außenfläche aufweist und die Innenfläche (3) durchlässig gegenüber den gasförmigen Aminen ist, die durch den Indikator (5) zu erfassen sind; wobei
die Form der Vorrichtung im Allgemeinen dreieckig ist, sodass Kanten (8) der Vorrichtung an den Leisten des Trägers anstoßen, wodurch unbeabsichtigte Bewegung verhindert wird; der Indikator entfernbar in einer Indikatortasche (6) platzierbar ist, die in der gasdurchlässigen Schicht der Innenfläche (3) bereitgestellt ist; und
der Indikator eine längliche Form aufweist und im Wesentlichen flach ist und aus einem weichen flexiblen Material gefertigt ist, das eine strukturelle Integrität aufweist, die sicherstellt, dass er einfach in die Tasche (6) eingeführt werden kann.

2. Vorrichtung nach Anspruch 1, wobei der chemichrome Farbstoff empfindlich gegenüber Aminen ist, die aus der Gruppe ausgewählt sind, die aus Trimethylamin, Putrescin und Cadaverin besteht.

3. Vorrichtung nach Anspruch 1, wobei chemichrome Farbstoffe aus der Gruppe ausgewählt sind, die aus Arylmethan-Farbstoffen besteht.

4. Vorrichtung nach Anspruch 3, wobei die Farbstoffe aus der Gruppe ausgewählt sind, die aus Diarylmethanen und Triarylmethanen besteht.

5. Vorrichtung nach einem der Ansprüche 1-4, nicht umfassend eine feuchtigkeitsabsorbierende Schicht.

6. Vorrichtung nach einem der Ansprüche 1-4, umfassend eine feuchtigkeitsabsorbierende Schicht (4) zwischen der Innenfläche (3) und der Flüssigkeitsbarriereschicht (2).

7. Vorrichtung nach einem der Ansprüche 1-6, ferner umfassend ein Band (7) quer über die Außenfläche der Vorrichtung, wobei das Band angeordnet ist, um einem Benutzer zu ermöglichen, die Vorrichtung zu halten, indem eine Hand zwischen das Band und den Rest der Vorrichtung eingefügt wird, ohne den Indikator zu berühren.

8. Vorrichtung nach einem der Ansprüche 1-7, ferner umfassend Mittel (9) zum entfernbaren Anbringen der Vorrichtung an einer Unterhose.

9. Vorrichtung nach einem der Ansprüche 1-8, ferner umfassend eine Tasche (10), die an der Innenfläche angebracht ist, um den Penis des Benutzers zu halten.

## Revendications

1. Dispositif de détection d'amines gazeuses provenant d'une maladie dans le système urogénital humain, ledit dispositif comprenant une surface extérieure (1) et une surface intérieure (3) destinées à faire face à l'utilisateur et à s'éloigner de l'utilisateur, respectivement, **caractérisé en ce que**
ledit dispositif comprend un indicateur (5) configuré pour détecter des amines gazeuses, comprenant des colorants chimio-chromiques sensibles aux amines qui changent de couleur en présence d'amines ;
ledit dispositif est constitué d'un matériau souple de forme adaptée pour recouvrir les orifices du système urogénital humain mais pas de l'anus, le dispositif ayant une barrière aux liquide (2) proche de la surface extérieure et la surface intérieure (3) est perméable aux amines gazeuses devant être détectées par l'indicateur (5) ; dans lequel
la forme du dispositif est généralement triangulaire de sorte que les bords (8) du dispositif viennent en butée contre l'aine du porteur, empêchant ainsi un mouvement indésirable ;
l'indicateur peut être placé de manière amovible dans une poche d'indicateur (6) prévue dans la couche perméable aux gaz de la surface intérieure (3) ; et
l'indicateur a une forme allongée et est essentiellement plat, et est fabriqué à partir d'un matériau souple doux ayant une intégrité structurelle lui permettant de pouvoir être facilement introduit dans la poche (6).

2. Dispositif selon la revendication 1, dans lequel le colorant chimio-chromique est sensible aux amines choisies dans le groupe constitué par la triméthylamine, la putrescine et la cadavérine.

3. Dispositif selon la revendication 1, dans lequel les colorants chimio-chromiques sont choisis dans le groupe constitué par les colorants arylméthane.

4. Dispositif selon la revendication 3, dans lequel les colorants sont choisis dans le groupe constitué par les diarylméthanes et les triarylméthanes.

5. Dispositif selon l'une quelconque des revendications 1 à 4, ne comprenant pas de couche absorbant l'humidité.

6. Dispositif selon l'une quelconque des revendications 1 à 4, comprenant une couche absorbant l'humidité (4) entre la surface intérieure (3) et la couche de barrière aux liquide (2).

7. Dispositif selon l'une quelconque des revendications 1 à 6, comprenant en outre une bande (7) à travers la surface extérieure du dispositif, dans lequel ladite bande est agencée pour permettre à un utilisateur de maintenir le dispositif par insertion d'une main entre la bande et le reste du dispositif, sans toucher l'indicateur.

8. Dispositif selon l'une quelconque des revendications 1 à 7, comprenant en outre un moyen (9) de fixer de manière amovible le dispositif à un sous-vêtement.

9. Dispositif selon l'une quelconque des revendications 1 à 8, comprenant en outre une poche (10) fixée à la surface intérieure, pour maintenir le pénis de l'utilisateur.
